# EUROPEAN PATENT APPLICATION

(11) **EP 4 755 424 A1**
(43) Date of publication of application: **10.06.2026**
(21) Application number: 23947424.0
(22) Date of filing: 20.12.2023
(51) Int. Cl.: A61M 11/00, A61M 11/04, A61M 15/00, A61M 15/08

(54) **ACCURATE DELIVERY DEVICE AND ACCURATE DRUG ADMINISTRATION CONTROL METHOD FOR DELIVERING PHARMACEUTICAL AEROSOL**

(30) Priority: 03.08.2023 CN 202310973051
(71) Applicant: Shi, Xiaoping, Taizhou, Zhejiang 317300 (CN)
(72) Inventor: SHI, Xiongwei, Taizhou, Zhejiang 317300 (CN)
(74) Representative: Chung, Hoi Kan
(86) International application number: PCT/CN2023/140088
(87) International publication number: WO 2025/025487

(57) **Abstract**

A precise delivery device for delivering pharmaceutical aerosol and a precision drug administration control method. The precise delivery device includes: a casing, the size and shape of which are configured to be held in a user's hand; a cartridge assembly capable of atomizing a set dose of drug into aerosol, the cartridge assembly having an aerosol channel therein for conveying the aerosol; a flow generator for generating a positive pressure airflow to deliver the aerosol to the respiratory tract; and a control unit for receiving operation instructions and driving the cartridge assembly to output drug aerosol and the flow generator to generate positive pressure airflow. The cartridge assembly, the flow generator, and the control unit are all accommodated within the casing.

## Description

### TECHNICAL FIELD

The present disclosure relates to the technical field of aerosolized drug delivery, and in particular, to a precise delivery device for pharmaceutical aerosol and a precision drug administration control method.

### BACKGROUND

Nasal/oral inhalation administration is a very special delivery way that can be applied to prevent and treat diseases of the central nervous system, respiratory system, and other aspects. (1) Studies have confirmed the existence of a direct anatomical pathway between brain tissue and the nasal cavity. Intranasal administration bypasses the blood-brain barrier to deliver drugs non-invasively to the brain directly. Specifically, drugs can reach the central nervous system through the nasal mucosa by two pathways: the olfactory epithelium or the respiratory epithelium, rapidly entering brain tissue and interacting with relevant receptors to exert therapeutic effects. The nose-to-brain connection can also function by activating peripheral nasal chemosensory neurons, triggering and modulating the olfactory-amygdala neural circuit, without the need for drugs to directly bind to neuronal receptors in the brain. This limits molecular transport into the circulatory system and minimizes potential systemic drug exposure. Regardless of the mechanism, its characteristics are bypassing the blood-brain barrier, the alveolar-blood barrier, hepatic first-pass effect, and gastrointestinal metabolism. Therefore, it requires the lowest dose (10⁻⁶-10⁻¹² g) with the fastest onset of action (within seconds), the least systemic toxicity and side effects, and the best compliance. (2) Studies have confirmed that pulmonary drug delivery is also an excellent route of administration. The characteristics of lung-targeted drug delivery are: a large absorption surface area, a rich capillary network, and a very thin alveolar-blood-air barrier, which avoids gastrointestinal metabolism and hepatic first-pass effect. Compared to oral and injectable administration, it requires lower doses, has a faster onset of action, causes fewer systemic toxic side effects, and offers better compliance. (3) Studies have confirmed that other drugs not targeting the central nervous system or respiratory system can also achieve better preventive and therapeutic effects through the nasal/oral inhalation administration route. Examples include peptide drugs for diabetes requiring frequent injections, osteoporosis drugs, hormonal drugs, and other oral or injectable drugs requiring rapid onset. Reformulating them into inhalation dosage forms to meet user's needs holds significant practical value.

Aerosolized inhalation dosage forms refer to formulations where the drug is administered via a specialized device, inhaled into deep parts of the respiratory tract, body cavities, mucous membranes, etc., to exert local or systemic therapeutic effects, thereby achieving the goal of painless, rapid, and effective treatment.

Formulations used for pulmonary administration may be further categorized into nebulizer solutions, metered-dose inhalers, dry powder inhalers, and soft mist inhalers. As a specific dosage form for pulmonary administration, nebulizer solutions are typically aqueous solutions. These solutions are converted into aerosols for user inhalation through specific devices. The inhaled dose deposits at different lesion sites within the respiratory tract at a certain rate and with appropriate particle sizes. The particle size of the aerosol is a primary factor affecting the deposition site in respiratory tract and has been widely studied. Another factor is the user's inhalation force and breathing habits. The same aerosolized agent and device can result in different inhaled doses and drug deposition sites in different users, leading to varying therapeutic outcomes. Metered-dose aerosols, dry powder inhalers, and soft mist inhalers also face the same issue of imprecise aerosolized dose, inhaled dose, and deposition site.

However, in the related art, existing aerosol inhalation devices typically include a nebulizer device, a solution storage device, an aerosol output tube, etc., and some even require an oxygen storage device. They are relatively complex, bulky, and expensive with inconvenient maintenance. They are generally not easy to transport or carry, only suitable for use in fixed locations such as hospitals, and are incapable of precise nebulization, inhalation, and deposition.

### BRIEF DESCRIPTION OF THE DRAWINGS

To illustrate the technical solutions of the embodiments of the present disclosure more clearly, the drawings of the embodiments will be briefly introduced below. Obviously, the drawings in the following description relate only to some embodiments of the present disclosure and are not intended to limit the present disclosure.
FIG. 1 is a schematic structural diagram of a closed-cover state according to Embodiment 1.
FIG. 2 is a schematic structural diagram of an open-cover state according to Embodiment 1.
FIG. 3 is an exploded view of FIG. 1.
FIG. 4 is a cross-sectional view according to Embodiment 1.
FIG. 5 is a view of the internal structure according to Embodiment 1.
FIG. 6 is a schematic structural diagram of a drug cartridge according to Embodiment 1.
FIG. 7 is a cross-sectional view of FIG. 6.
FIG. 8 is an exploded view of FIG. 6.
FIG. 9 is a distribution diagram of aerosol particles output by the drug cartridge.

Reference Numerals: 1. Box cover; 2. Casing; 2a. Left shell; 2b. Right shell; 2c. Air inlet hole; 3. Indicator light; 4. Button; 5. Drug cartridge; 5a. Cartridge shell; 5a1. Aerosol outlet nozzle; 5b. Solution retaining part; 5c. Aerosol channel; 5d. Atomizer; 5e. Base; 6. Drug cartridge sleeve; 7. Airflow gathering hood; 8. Flow generator; 9. Circuit board; 10. Battery.

### DETAILED DESCRIPTION

To make the objectives, technical solutions, and advantages of the embodiments in the present disclosure clearer, the present disclosure will be further described in detail below with reference to the accompanying drawings. Components of the embodiments of the present disclosure, which are generally described and illustrated in the drawings herein, may be arranged and designed in a variety of different configurations. Based on the embodiments of the present disclosure, all other embodiments obtained by those skilled in the art without making creative efforts shall fall within the scope of the present disclosure.

The implementations of the present disclosure are described in detail below with reference to the accompanying drawings. Features in the following embodiments may be combined with each other if not conflicting.

### Embodiment 1

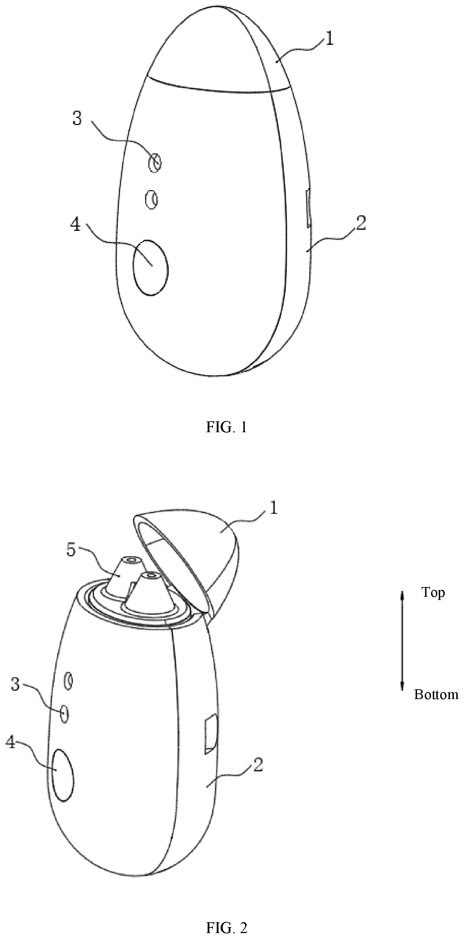

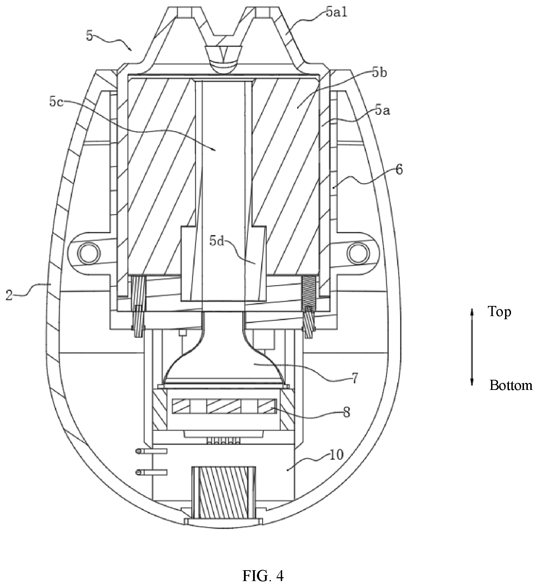

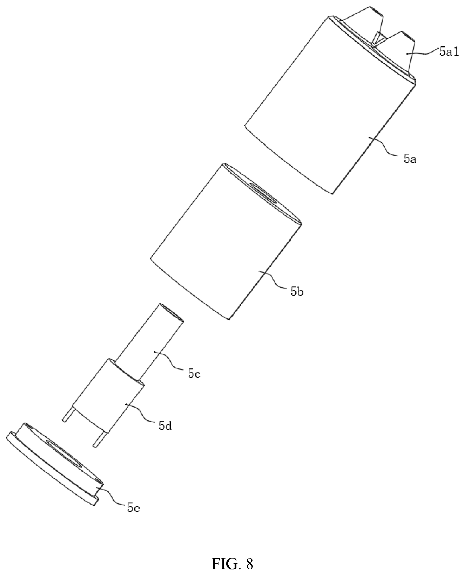

Referring to FIGS. 1-9, the present disclosure provides a precise delivery device for delivering pharmaceutical aerosol, including a casing 2, a drug cartridge 5, a flow generator 8, and a control unit. The size and shape of the casing 2 are configured to be held in a user's hand. The drug cartridge 5 is capable of atomizing a set dose of a drug into an aerosol. The drug cartridge 5 has an aerosol channel 5c arranged in the drug cartridge 5 for conveying the aerosol. The flow generator 8 is configured to generate a positive-pressure airflow for delivering the aerosol to the respiratory tract, which includes the mouth/nose, trachea, and lungs, and the aerosol enters the trachea and lungs via the mouth/nose. The velocity of this positive-pressure airflow may be set and adjusted to accurately deliver the aerosol to the user's regional deposition, achieving targeted drug delivery and significantly improving drug utilization and therapeutic effect. The control unit is configured to receive user operation instructions, and to drive the drug cartridge 5 to output the pharmaceutical aerosol, while simultaneously driving the flow generator 8 to generate a set amount of positive-pressure airflow to drive the aerosol into the respiratory system. The drug cartridge 5, the flow generator 8, and the control unit are all accommodated within the casing 2.

The aforementioned precise delivery device can actively and targetedly deliver the pharmaceutical aerosol to the user's respiratory system, revolutionizing the long-standing problems of aerosol inhalation. That is, the inhaled dose of the drug and the deposition location of the drug depend on the user's lung capacity, breathing force, inhalation technique, and the particle size of the aerosol. Active atomization and delivery may solve the problem for some users, especially chronic pulmonary disease patients, the elderly, and children with relatively weak breathing force, who cannot generate sufficient breathing force to inhale the drug. It may further solve the problem of self-administration for rescue purposes, such as for some drug overdose users who are in a weak state (e.g., in shock) during emergency episodes of diseases like type I allergic reactions leading to dyspnea. In addition, this delivery device has the characteristic of being compact and small, and can be held in the user's hand. Therefore, this aerosol delivery device has the advantages of being portable and convenient to use, and does not require hospitalization, saving medical resources and significantly improving user compliance. Through active, precise atomization and precise delivery, the present disclosure can achieve partial or full respiratory tract targeted deposition of the drug, thereby reducing harm caused by the drug to non-lesion areas.

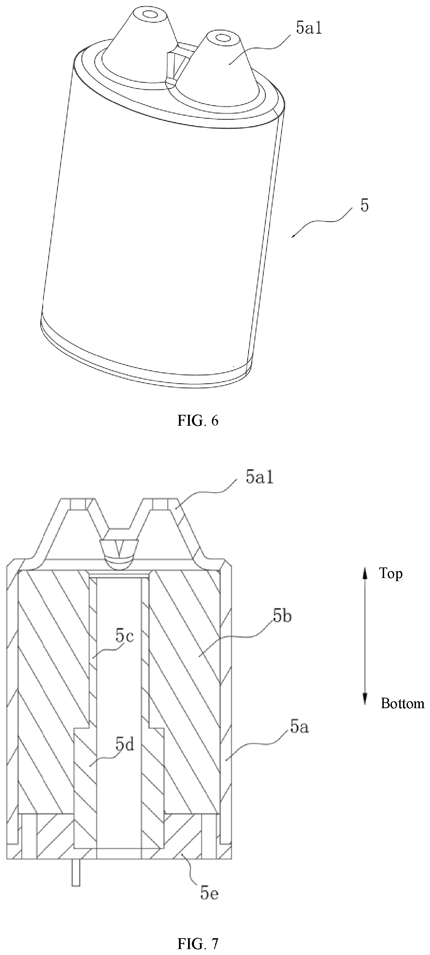

As shown in FIGS. 6-8, the drug cartridge 5 includes a cartridge shell 5a and an atomizer 5d. The interior of the cartridge shell 5a defines a drug chamber, and the atomizer 5d is positioned within the cartridge shell 5a to atomize the active pharmaceutical ingredient. In the embodiments, the drug is loaded into the drug chamber in solid or liquid dosage form. Specifically, the drug may be mixed with solid excipients to form a solid formulation (only suitable for heating atomization) or dissolved in a solvent to form an atomizable solution, which is then filled into the drug chamber. Alternatively, a solution retaining part 5b, such as a cotton wick, porous ceramic, adsorbent, or other liquid-absorbing porous materials, may be arranged within the drug chamber. To prevent loss of activity over time, the drug and the atomization solvent may be packaged separately, and a barrier membrane can be removed before use to dissolve the drug into the atomization solvent, thereby extending the drug's shelf life.

As shown in FIG. 7, to facilitate adding the drug solution or dissolving the drug into a solution, a base 5e may be attached to a bottom of the cartridge shell 5a. The base 5e and the cartridge shell 5a together define the drug chamber. Specifically, the base 5e may be a rubber or silicone part. The base 5e is detachably attached to the cartridge shell 5a and can be plugged into an opening at the bottom of the cartridge shell 5a to seal the opening, thereby providing a sealing effect and preventing drug solution leakage. In another implementation, the base 5e and the cartridge shell 5a may be set as an integrated part, or may be welded or fixed together in subsequent processes. The sealing of this structure is better than a split one, because it does not allow users to replace the drug solution, which can only be discarded after use, resulting in relatively higher cost but preventing cross-contamination.

Since the atomizer 5d is disposed inside the cartridge shell 5a, to supply electrical energy to the atomizer 5d, an electrode part for transmitting electrical energy to the atomizer 5d is embedded in the base 5e. Specifically, there are two electrode parts, which are metal pieces or other conductive objects embedded in the base 5e.

As shown in FIGS. 6-8, a top of the cartridge shell 5a has at least one upwardly extending aerosol outlet nozzle 5a1. Specifically, in the embodiments, two aerosol outlet nozzles 5a1 may be provided, one on the left and one on the right, corresponding to two nostrils. Therefore, the aerosol precise delivery device in the illustrated embodiments is mainly for nasal delivery, inducing the central nervous system through the nasal mucosa and the olfactory bulb to treat diseases related to mood and mental aspects. The delivery device of the present disclosure can actively deliver drugs according to user needs, thereby solving the problem for some users with insufficient breathing force who have difficulty actively inhaling an appropriate dose of drug and controlling the drug deposition site. For example, a user who has overdosed on drugs is often in a state of confusion, with clenched teeth and weak breathing, making oral and injectable administration impossible, and also impossible to use rescue drugs via active inhalation, thus losing valuable treatment opportunities. The aerosol precise delivery device in the embodiments may achieve accurate and timely treatment or self-rescue of the user through active targeted drug delivery (e.g., delivering antidote actively through the nasal cavity and respiratory tract). Awakening of post-operative anesthetized patients is usually only possible through injection; or during an emergency episode of a type I allergic reaction causing dyspnea, patients usually self-administer backup drugs via injection. The aerosol precise delivery device in the embodiments, through active nasal and respiratory tract administration, may completely replace injection administration. The outlet diameter D of the aerosol outlet nozzle 5a1 satisfies 1.0mm ≤ D ≤ 3.5mm. The aerosol outlet nozzle 5a1 within this diameter range may achieve a balance between aerosol output amount and output velocity, avoiding the problem of air trapping caused by an overly small outlet nozzle 5a1, while a larger outlet nozzle 5a1 would result in insufficient aerosol force, failing to effectively deliver the drug to the user. Moreover, the above diameter setting may ensure the aerosol outlet nozzle 5a1 fits the nasal cavity, thereby improving comfort during use. Specifically, the diameter D of the aerosol outlet nozzle 5a1 is 2.5mm, or it may be any value within the range of 1.0mm ≤ D ≤ 3.5mm. The inner surface of the aerosol outlet nozzle 5a1 is configured as a cone shape capable of converging the aerosol, facilitating fit with the nostril, preventing leakage, and improving the delivery accuracy of the aerosol.

As shown in FIGS. 6 and 7, the aerosol outlet nozzle 5a1 and the atomizer 5d are in communication via an aerosol outlet tube. The function of the aerosol outlet tube is to guide the generated aerosol upward to the aerosol outlet nozzle 5a1 to prevent air trapping. Specifically, a pipe may be connected to the atomizer 5d, and the generated aerosol can flow into the aerosol outlet nozzle 5a1 through this aerosol outlet tube. Specifically, the aerosol outlet tube may be a metal tube extending from above the atomizer 5d to below the aerosol outlet nozzle 5a1. In the present disclosure, the aforementioned air trapping refers to a situation where the outlet flow rate is less than the aerosol production rate, causing the aerosol to fail to flow out effectively, seemingly trapped within the aerosol channel 5c, thereby reducing delivery accuracy.

In the embodiments, the casing 2 may include a drug cartridge sleeve 6 for accommodating the drug cartridge 5. The drug cartridge 5 is detachably assembled within the drug cartridge sleeve 6, facilitating user replacement of the drug cartridge 5. For users, being able to conveniently replace the drug cartridge 5 is very necessary, which allows users to use the same main body to replace drug cartridges with different drugs for atomization administration, reducing costs.

It should be noted that the atomizer 5d in the present disclosure may be a heating atomization device or an ultrasonic atomization device. For drugs with excellent thermal stability, the drug solid preparation may be directly heated or prepared into a liquid atomization agent and then heated, causing it to evaporate and atomize into an aerosol. Since some drugs are unstable at high temperatures and prone to decomposition and inactivation, ultrasonic atomization devices are more advantageous than heating atomization devices for thermally unstable drugs. The heating atomization device is a heating element (for solid atomizing agents), ceramic core, or cotton wick heating atomizer (for liquid atomizing agents), and similar heating atomization methods are already widely applied in the electronic cigarette industry. The ultrasonic atomization device is a piezoelectric ceramic atomizer or mesh atomizer. Ultrasonic atomization is also a conventional atomization method, widely used in the medical and beauty industries. Its specific structure will not be described in detail herein. Furthermore, the flow generator 8 in the present disclosure may be a fan or an air pump. A fan with adjustable air volume and wind speed is preferred. For the air pump, a micro air pump produced by Murata Manufacturing Co., Ltd. may be selected, whose flow rate can be adjusted by changing the current value. Specifically, the flow generator 8 in the present embodiments may be a fan. It should be further noted that in order to introduce the solution into the atomizer 5d for atomization, for a heating-type atomizer 5d, a liquid guiding member is usually arranged around the atomizer 5d to guide the solution to the vicinity of the atomizer 5d. During heating, the solution within the liquid guiding member is directly atomized into aerosol. The liquid guiding member is usually made of porous, temperature-resistant materials, such as ceramic, cotton fiber, etc.

In order to enable the aerosol to be rapidly expelled from the aerosol outlet nozzle 5a1, a bottom of the drug cartridge sleeve 6 in the embodiments may be arranged with an upper air inlet. The air outlet of the fan is in communication with the upper air inlet, and the airflow generated by the fan enters the drug cartridge 5 via the upper air inlet. An airflow gathering hood 7 is arranged between the flow generator 8 and the drug cartridge sleeve 6 for gathering and guiding the airflow into the drug cartridge 5. The airflow gathering hood 7 includes an air outlet pipe extending into the drug cartridge sleeve 6, and the air outlet pipe is connected to the upper air inlet of the drug cartridge 5. In another implementation, to more efficiently guide the airflow generated by the fan into the drug cartridge 5, the airflow gathering hood 7 defines a conical inner cavity for gathering airflow. That is, the inner cavity of the airflow gathering hood 7 is designed as a conical inner cavity, and the airflow can be gathered and then enter the drug cartridge 5 from the top of the conical inner cavity.

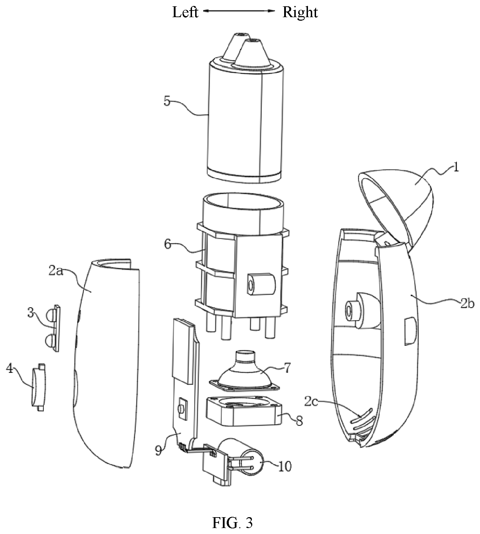

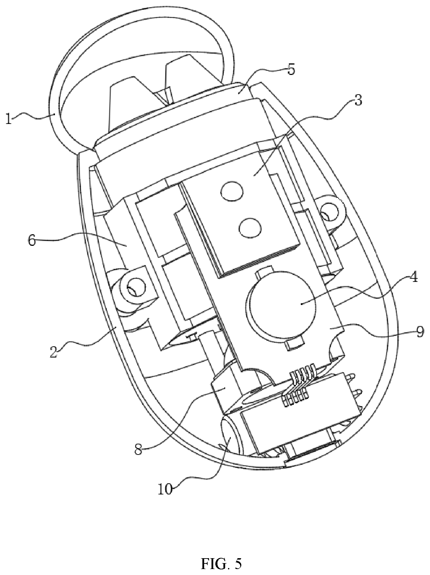

As shown in FIGS. 3 and 5, the control unit includes a circuit board 9. An indicator light 3 and a button 4 are disposed on the circuit board 9. To save space, the circuit board 9 is arranged vertically in a gap between the cartridge shell 5a and the casing 2. This configuration may significantly reduce the overall size of the device, making the product easy to hold in the user's palm and convenient for user operation. The casing 2 incorporates an operation part for the user to press. The fan and the cartridge shell 5a press against the back of the circuit board 9 to support the operation part, thereby reducing installation difficulty and saving internal space. When the user places the product in a pocket, handbag, or backpack, mutual squeezing between items may easily cause accidental activation leading to waste, and may contaminate the user's other items. To prevent accidental activation, in the embodiments, the surface of the button 4 may be flush with or slightly lower than the outer surface of the casing 2.

To further reduce the overall size of the product, the battery 10 may be placed horizontally in an air inlet chamber. Specifically, the air inlet chamber is defined between the fan and the casing 2, and the battery 10 is arranged in the air inlet chamber. To facilitate installation of the internal structure, the casing 2 is divided into a left shell 2a and a right shell 2b. The top of the casing 2 has an opening. The drug cartridge sleeve 6 is clamped between the left shell 2a and the right shell 2b. The drug cartridge 5 is slidably installed within the drug cartridge sleeve 6 in the up-down direction. FIG. 5 shows the internal structure view after removing the left shell 2a. The left shell 2a and the right shell 2b can be fixedly installed using fasteners, such as screws, or can be fixedly installed using clips. An air inlet hole 2c is defined on the right shell 2b. Additionally, a box cover 1 is connected to the top of the casing 2. The box cover 1 may be pivotally connected to the casing 2. Flipping the box cover 1 allows for closing and opening, which is convenient and hygienic to use.

The beneficial effects produced by the above technical solutions are as follows.

The drug precise delivery device of the present disclosure can actively and targetedly deliver pharmaceutical aerosol to the user's respiratory system, revolutionizing the long-standing problems of aerosol inhalation drug administration. That is, the inhaled dose of the drug and the deposition location of the drug depend on the user's lung capacity, breathing force, inhalation technique, and the particle size of the aerosol. Active atomization and delivery solves the problem for some users, especially long-term pulmonary disease patients, the elderly, and children with relatively weak breathing force, who cannot generate sufficient breathing force to inhale the drug. It may further solve the problem of self-administration for rescue purposes for some drug overdose users who are in a weak state due to excessive drug use or during emergency episodes of diseases like type I allergic reactions leading to dyspnea. In addition, this delivery device has the characteristic of being compact and small, and can be held in the user's hand. Therefore, this aerosol delivery device has the advantages of being portable and convenient to use. Through active atomization and delivery, the present disclosure can achieve partial or full respiratory tract targeted deposition and administration of the drug, thereby significantly improving the drug's efficacy and reducing side effects of the drug on the liver and other organs.

### Embodiment 2

Referring to FIGS. 1-8, the present disclosure further provides a precision drug administration control method for delivering aerosol via positive pressure airflow, applicable to the aforementioned precise delivery device. The precision drug administration control method includes:
controlling the drug cartridge 5 to operate for a set duration to generate a pharmaceutical aerosol; and
controlling the operation of a fan to generate a positive pressure airflow to expel the aerosol from the atomization chamber.

In some embodiments, the drug cartridge 5 may be controlled to operate for a set duration to generate the pharmaceutical aerosol, and then the fan may be further controlled to operate to generate the positive pressure airflow to expel the aerosol from the atomization chamber into the respiratory tract. Specifically, in the embodiments, the set duration may be 2.5s, and it may be other time values selected from the range of 0.2~5s, for example, 1s, 2s, or 3.5s. A person skilled in the art can select an appropriate set duration after sufficient laboratory verification. The atomizer 5d may be a heating-type atomizer 5d, meaning the aerosol is generated first, and then the airflow is generated to expel the aerosol, thereby preventing the airflow from reducing the temperature of the atomizer 5d, which would affect the atomization effect. In another implementation, the drug cartridge 5 and the fan may be controlled to work simultaneously, meaning the airflow and aerosol are generated concurrently. In the embodiments, to achieve precise drug administration, the dose of the aerosol may be controlled by the operating time and power of the atomizer 5d, while simultaneously controlling the air volume and velocity to accurately deliver the drug to the required deposition site within the respiratory tract, thereby achieving targeted drug delivery.

In addition, the precision drug administration control method may further include: acquiring an AI prescription set by a doctor based on user information. The AI prescription includes the timing, frequency, interval, intensity, and dose of atomized drug administration. The control unit controls the operation of the drug cartridge 5 and the flow generator 8 according to the AI prescription. Specifically, the AI prescription is formulated based on drug concentration, number of atomizations, atomization interval, atomization duration, atomization power, and user-specific information, thereby enabling intelligent, precise, and personalized targeted drug administration. The aforementioned user information may include the user's disease diagnosis and treatment history, drug allergy history, health constitution information, genetic information, etc. Administering drug precisely based on user information allows for better diagnosis and treatment, achieving optimal therapeutic effects.

Each drug atomization and delivery operation of the precision atomization device involved in the present disclosure is equivalent to the pulse theory in electromagnetics, and the terminology is borrowed herein. Precision pulsed atomization administration parameters include pulse length (pulse width), number of pulses (pulse frequency), interval (pulse period), amplitude (pulse peak height), and energy (pulse peak area), which are set as corresponding atomization parameters in the precise administration "AI prescription": atomization duration, administration frequency, administration interval, administration intensity, and administration dose.

The start time node, pulse duration (width), pulse frequency (number), pulse interval (period), pulse amplitude (peak height), and pulse energy (peak area) of the applied pulses may be synchronized with the user's inhalation start time, inhalation duration, number of puffs, interval between puffs, inhalation force, and inhalation dose. Alternatively, the administration time node, duration, number, interval, force, and dose may be preset via a manual button or program to implement timed and quantified precise administration.
(a) The pulse period may be divided into long period and short period. For example, when the average daily medication usage is 3 times, each administration requires delivering 5 times or 5 puffs (5 pulse doses), each taking 2.5 minutes, then the long period is about 24/3 = 8 hours, and the short period is about 2.5/5 = 0.5 minute (i.e., 30s).
(b) Drug dose includes: (1) The dose of atomized solution per pulse and its active ingredient per pulse (pulse dose); (2) The total dose of atomized solution and total active drug per administration (cumulative total of multiple pulses per administration); (3) The total daily dose of atomized solution and total active drug (cumulative total of multiple administrations per day).
(c) These parameters may be programmed by an intelligent "AI doctor" to create a "tailor-made" drug administration "AI prescription" program based on drug concentration, number of atomizations, atomization duration, timing, output power, and user-specific situation, which is then implanted into the user's atomization device. This determines the user's medication time node for each administration, number of deliveries, dose per pulse, total number of pulses and total dose per administration, total daily inhalation frequency, and total daily inhalation dose.
(d) The user strictly follows the medication program set by the "AI prescription". The chip program instantly records medication usage and therapeutic effects, and the data is immediately fed back to the "AI doctor" and "AI medical record" archive. The "AI doctor" remotely monitors the user's medication usage and therapeutic effects, and can modify administration parameters and adjust the "AI prescription" at any time. The atomization device then administers the drug according to the latest control program instructions. In addition, the atomization device or cloud establishes the user's "AI medical record" archive, receiving and storing the user's immediate usage data, therapeutic effects, and various adverse reactions, and promptly feeding these back to the "AI doctor".
(e) Completely different from fixed-specification (dose) dosage forms such as tablets, capsules, patches, and injectables, theoretically, intelligent atomized drug administration can, by modifying the program, adjust the atomization node, atomization duration, number of atomizations, atomization duration, and atomization dose at any time, dynamically changing the drug dose "specification" and frequency of administration. This allows the dose to be precisely divided, always keeping the user's drug concentration in the optimal state to achieve the best therapeutic effect.
(f) The intelligent "AI doctor" can issue a dynamic, personalized "AI prescription" based on the user's genetic information, ethnicity, gender, age, weight, height, medical history, and the latest collected test, evaluation, and medication feedback data from the user's "AI medical record" archive. Upon receiving the new "AI prescription", the intelligent atomization device strictly executes the programmed atomized drug administration, achieving the goal of program-controlled, data-driven administration. In addition, it continuously collects medication and efficacy data, feeds them back to the "AI doctor", and continuously enriches the "AI medical record" archive data, thereby forming a dynamic closed loop.

The present disclosure successfully introduces the concept of pulses from electromagnetic theory, organically correlating and corresponding parameters such as pulse duration (peak width), number (frequency), interval (period), amplitude (peak height), and energy (peak area) with atomized administration parameters like administration duration, frequency, interval, intensity, and dose. This derivation gives rise to the concept of an "AI doctor", further leading to the concepts of an "AI prescription" and an "AI medical record" archive. Defining these basic parameters, combined with the characteristics of precisely divisible atomized solution and the instant variability of the "specification" of liquid atomized agents, lays a solid foundation for intelligent, precise, and personalized drug administration.

Before the true emergence of an intelligent "AI doctor", intelligent atomization inhalation devices can utilize electronic monitoring systems connected to the internet or other devices, either externally or through integration, to improve user's medication compliance and reduce medication errors caused by the device or operator, especially for some controlled drug substances. External intelligent systems with comprehensive monitoring and feedback functions can record user's medication information, enabling data sharing between doctors and patients, allowing doctors to adjust treatment plans promptly. Besides having functions like reminders, recording, and management, they can also upload information to mobile phones via Bluetooth. The self-integrated intelligent atomizer 5d adopts adaptive atomization delivery technology, determining the atomized drug transmission time during inhalation by analyzing the user's breathing pattern.

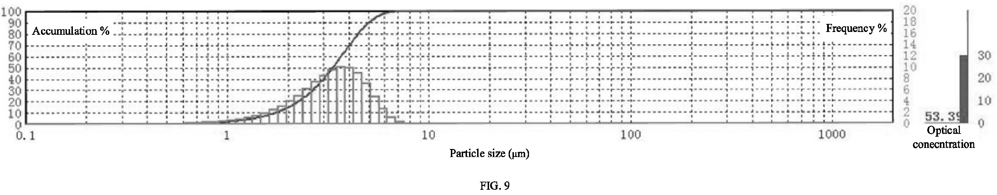

Furthermore, to achieve precise drug administration, the drug cartridge 5 of the present disclosure is configured to controllably output atomized particles with a size range of 20nm to 20µm. Aerosols of different particle sizes deposit in corresponding parts of the respiratory tract, enabling partial or full respiratory tract targeted administration. The respiratory tract is divided into upper and lower parts: the nose, pharynx, and larynx are collectively called the upper respiratory tract. The trachea, bronchi, and lung organs are collectively called the lower respiratory tract, or the tracheobronchial tree. Based on the Applicant's research, atomized particles of different sizes deposit at different locations in the respiratory tract. By controlling the particle size of the atomized particles, different respiratory tract locations can be targeted for drug administration, thus achieving precise delivery. For a heating-type cotton wick atomizer 5d, the size of the atomized particles may be adjusted by controlling the viscosity of the atomizing agent, the power of the heating element, and the airflow. For a ceramic core-type atomizer 5d, the particle size may be adjusted by controlling the viscosity of the atomizing agent, the pore size of the porous ceramic, the power of the heating element, and the airflow velocity. However, for ultrasonic atomizer wafers, the particle size of the atomized particles must be controlled by a mesh screen. FIG. 9 is a distribution diagram of aerosol particles output by the drug cartridge 5 measured by professional instrument equipment. As shown in FIG. 9, the bar graph as illustrated indicates the probability of aerosol occurrence within different particle size ranges, and the wavy line indicates the percentage of aerosol quantity within that particle size range. It can be seen that the particle size range of the atomized particles generated by the atomization device in this example is roughly 1~10µm, with nearly 100% of the aerosol quantity falling within this range, approximately following a normal distribution. Furthermore, particles in the 1~5µm size range constitute about 60%, not only ensuring the stability of particle size output but also enabling these particles to deposit in the patient's lungs, thereby achieving precise drug administration and improving drug therapeutic efficacy. It should be noted that the aforementioned professional instrument equipment may be a PSA series laser particle size analyzer produced by Anton Paar. This analyzer utilizes Dynamic Light Scattering (DLS) technology for measurement, a physical technique configured to characterize the size of particles and molecules in suspensions or solutions.

Additionally, the drug cartridge 5 is configured to controllably adjust the output amount of the aerosol. Specifically, the output amount of the aerosol may be adjusted by controlling the power of the atomizer 5d, the atomization duration, and the air volume of the fan. The drug cartridge 5 is configured such that the output flow rate of the aerosol is 1.0~50 mL/s. In some embodiments, the output flow rate of the aerosol is 8 mL/s. This adjustment of the output amount may be programmatically set based on the aforementioned AI prescription, or it may be manually adjusted based on the user's actual drug requirement. An adjustment device, such as a knob or screen, may be arranged on the device for the user to adjust the aerosol output. That is, the control unit includes a dose adjustment part that is operably adjustable for aerosol output.

Furthermore, for precise administration, an identification device for labeling administration information may be arranged on the drug cartridge 5, and the control unit can may configured to acquire the administration information and set the aerosol output per second and output duration of the drug cartridge 5 based on this information. For example, the identification device may be an RFID, NFC, barcode, etc. The administration information is written into the identification device, and the control unit has a corresponding reading device to read the aforementioned administration information, thereby controlling the aerosol output per second and output duration of the drug cartridge 5 according to this information. The aforementioned control may be further combined with the AI prescription to achieve a more precise method of administration.

It should be added that after the controlled shutdown of the drug cartridge 5, the control unit controls the flow generator 8 to shut down with a delay according to the set administration location, to generate an incremental airflow to deliver the aerosol to the administration location in the respiratory tract. The delay time range is 0.1~5.0s, specifically it may be 0.2s, 0.3s, 0.5s, etc., which may be set according to the targeted administration location. Within a certain time range, longer operation time of the flow generator 8 can deliver the aerosol to deeper parts of the respiratory tract.

The aforementioned precision administration method may be implemented in the following manner.
(1) The atomization device (drug cartridge + flow generator + control unit) is set to generate 100 pulses per gram of atomized solution (100 pulses/gram of atomized solution).
(2) The atomization duration is set to 2.5 seconds (i.e., pulse width = 2.5 seconds).
(3) Fan air delivery duration is 3.0 seconds (delay 0.5 seconds), air delivery rate is 8 mL/second. The total volume delivered to the mouth/nose each time = (2.5 x 8) + (0.5 x 8) = 24 mL. The first 20 mL of air delivery occurs concurrently with atomization, the last 4 mL is air only, pushing the residual aerosol in the airway into the mouth/nasal cavity.
(4) For nasal administration, the delivered 24 mL aerosol precisely fills the entire nasal cavity (average nasal cavity volume = 24 mL). At this time, the two aerosol outlets fit tightly against the two nostrils with no gap, and breath is held for 5-10 seconds. The aerosol deposits on the nasal mucosa, eliminating the need for separate sprays in the left and right nostrils.
(5) When it is necessary that the administration is via oral inhalation while simultaneously activating atomization and air delivery (with a microphone switch, i.e., gas-sensitive switch, installed), the 24 mL aerosol delivered to the mouth may be followed by continued inhalation or continued air delivery (i.e., inhalation/air delivery time ≥ 3.0 seconds), after which breath is held for 5-10 seconds. The aerosol will deposit at different lesion sites in the oral cavity, throat, upper respiratory tract, and lower respiratory tract. The specific deposition site depends on the duration of inhalation/air delivery and the length of the air delivery delay.
(6) The dose of atomized solution per pulse is 10 mg (i.e., set 100 pulses/gram of atomized solution).
(7) Each administration involves N pulses, with a pulse interval of 30 seconds (i.e., set short pulse period = 30 seconds, N=1-20).
(8) Administer M times per day (i.e., set long pulse period = 24/M hours).
(9) Atomized solution dose per pulse = 10 mg/pulse; total atomized solution dose per administration = N × 10 mg; total daily atomized solution dose = M × N × 10 mg.
(10) When the drug concentration in the atomized solution = X%, the active drug dose per pulse = 10 mg × X%, total active drug dose per administration = N × 10 mg × X%, and total daily active drug dose = M × N × 10 mg × X%.

The above 1.0g (100 pulses) of drug atomized solution (10%) is loaded into the drug chamber of the "heated atomization (ceramic core)" or "ultrasonic atomization (mesh vibration)" drug cartridge of the present disclosure. The control unit program controls nasal/oral inhalation pulse administration 3 times/day, 5 pulses each time, each atomization lasting 2.5 seconds, with atomization every 30 seconds (2 pulses/minute); dose per inhalation = 10 mg/pulse; fan air delivery duration 3.0 seconds (delay 0.5 seconds), air delivery rate 8 mL/second.

Corresponding AI prescription parameters: daily pulse count = 3 × 5; pulse width 2.5 seconds; short period = 30 seconds; long period I = 24/3 hours (disregarding sleep intervals); atomized solution dose per inhalation = 10 mg/pulse; total atomized solution dose per administration = 5 × 10 mg/time; total daily atomized solution dose = 3 × 5 × 10 mg/day; active drug dose per inhalation = 10 mg × 10%/pulse; total active drug dose per administration = 5 × 10 mg × 10%/time; total daily active drug dose = 3 × 5 × 10 mg × 10%/day.

The aforementioned atomization AI prescription parameters (drug cartridge administration information) is input or read into the control unit. The atomization device will strictly follow the set AI prescription, promptly remind the patient of administration times, accurately provide the drug dose for each pulse, the number of pulses per inhalation, and the daily inhalation frequency, thereby executing intelligent, precise, and personalized atomized drug administration.

According to the above technical solutions, the present disclosure may achieve intelligent and personalized atomized drug administration. An attending physician or an AI doctor can formulate a personalized administration method, i.e., an AI prescription, based on the user's individual situation. The AI prescription is then transmitted to the precise delivery device and saved. The control unit executes the administration program according to the AI prescription, providing personalized treatment for the user, which may significantly improve the optimal therapeutic effect for each user. Additionally, the present disclosure may further achieve the precise delivery of a precisely dosed drug to the user's treatment site, thereby achieving the goal of precise treatment to improve the drug's therapeutic efficacy and reduce its toxic side effects.

The above descriptions are merely specific implementations of the present disclosure. However, the scope of the present disclosure is not limited thereto. Those skilled in the art, within the technical scope disclosed by the present disclosure, may easily think of changes or substitutions, which should be covered within the scope of the present disclosure. Therefore, the scope of the present disclosure shall be subject to the protection scope of the claims.

## Claims

1. A precise delivery device for delivering pharmaceutical aerosol, comprising:
a casing, adapted to be held in a hand of a user;
a drug cartridge, configured to atomize a set dose of drug into aerosol and having an aerosol channel inside for conveying the aerosol;
a flow generator, configured to generate a positive pressure airflow to deliver the aerosol to a respiratory tract of the user; and
a control unit, configured to drive the drug cartridge to output the aerosol and drive the flow generator to generate a set amount of the positive pressure airflow according to an operation instruction;
wherein the drug cartridge, the flow generator, and the control unit are all accommodated within the casing.

2. The precise delivery device according to claim 1, wherein the drug cartridge comprises:
a cartridge shell; wherein an interior of the cartridge shell defines a drug chamber; and
an atomizer, positioned within the cartridge shell to atomize the drug.

3. The precise delivery device according to claim 2, wherein a base is attached to a bottom of the cartridge shell, and the base and the cartridge shell together define the drug chamber.

4. The precise delivery device according to claim 3, wherein the base is detachably attached to the cartridge shell.

5. The precise delivery device according to claim 3 or 4, wherein an electrode part is embedded in the base for transmitting electrical energy to the atomizer.

6. The precise delivery device according to claim 2, wherein a top of the cartridge shell is arranged with at least one atomization nozzle extending upward.

7. The precise delivery device according to claim 6, wherein an inner surface of the at least one atomization nozzle is configured as a cone shape capable of converging the aerosol.

8. The precise delivery device according to claim 6, wherein the at least one atomization nozzle is in communication with the atomizer via the aerosol channel.

9. The precise delivery device according to claim 1 or 2, wherein the casing comprises a drug cartridge sleeve for accommodating the drug cartridge; the drug cartridge is detachably assembled in the drug cartridge sleeve.

10. The precise delivery device according to claim 9, wherein a top air inlet is arranged on a bottom of the drug cartridge sleeve, and the flow generator is in communication with the top air inlet.

11. The precise delivery device according to claim 10, wherein an airflow gathering hood for gathering and guiding airflow into the drug cartridge is arranged between the flow generator and the drug cartridge sleeve.

12. The precise delivery device according to claim 6, wherein the airflow gathering hood comprises an air outlet pipe extending into the drug cartridge sleeve; the air outlet pipe is connected to the top air inlet of the drug cartridge.

13. The precise delivery device according to claim 11 or 12, wherein the airflow gathering hood defines a conical inner cavity for gathering airflow.

14. The precise delivery device according to claim 2, wherein the atomizer is a heating atomizer or an ultrasonic atomizer.

15. The precise delivery device according to claim 2, wherein the heating atomizer is a ceramic core heating atomizer or a cotton core heating atomizer, and the ultrasonic atomizer is a piezoelectric ceramic atomizer or a mesh screen vibration atomizer.

16. The precise delivery device according to claim 1, wherein the flow generator is a fan or an air pump.

17. The precise delivery device according to claim 1, wherein the control unit comprises a circuit board, and the circuit board is arranged vertically in a gap between the casing and the drug cartridge.

18. The precise delivery device according to claim 17, wherein the casing comprises an operation part,the flow generator is pressed against a back of the circuit board to support the operation part.

19. The precise delivery device according to claim 1, wherein the drug cartridge is configured such that an output flow rate of the aerosol is 1.0~50 mL/s, optionally , the output flow rate is 8 mL/s.

20. The precise delivery device according to claim 19, wherein an output duration of the aerosol is 0.2~5.0 s/time, optionally, the output duration is 2.5 s/time.

21. The precise delivery device according to claim 1, wherein an air inlet chamber is defined between the flow generator and the casing, and a battery is installed in the air inlet chamber.

22. The precise delivery device according to claim 21, wherein the battery is placed horizontally in the air inlet chamber.

23. The precise delivery device according to claim 1, wherein the set amount of the positive pressure airflow has parameters of flow rate and duration.

24. A precision drug administration control method for delivering aerosol via positive pressure airflow, applicable to the precise delivery device according to any one of claims 1~23,wherein the precise delivery device comprises:
a casing, adapted to be held in a hand of a user;
a drug cartridge, configured to atomize a set dose of drug into aerosol and having an aerosol channel inside for conveying the aerosol;
a flow generator, configured to generate a positive pressure airflow to deliver the aerosol to a respiratory tract of the user; and
a control unit, configured to drive the drug cartridge to output the aerosol and drive the flow generator to generate a set amount of the positive pressure airflow according to an operation instruction;
wherein the drug cartridge, the flow generator, and the control unit are all accommodated within the casing;
wherein the precision drug administration control method comprises:
controlling the drug cartridge to operate for a set time to generate the aerosol; and
controlling the flow generator to operate to generate the set amount of the positive pressure airflow to discharge the aerosol from the aerosol channel.

25. The precision drug administration control method according to claim 24, further comprising:
obtaining an AI prescription based on user information; wherein the AI prescription comprises: atomization administration duration, administration frequency, administration interval, administration intensity, and administration dose; and
controlling, by the control unit, an operation of the drug cartridge and the flow generator according to the AI prescription.

26. The precision drug administration control method according to claim 25, wherein the AI prescription is formulated based on drug concentration, number of atomizations, atomization duration, atomization power, and user information.

27. The precision drug administration control method according to claim 26 wherein the drug cartridge is configured to controllably output atomized particles with a particle size range of 20 nm to 20 µm, to implement partial or entire respiratory tract administration.

28. The precision drug administration control method according to claim 24, wherein the drug cartridge is capable of controllably adjusting an output dose of the aerosol.

29. The precision drug administration control method according to claim 28, wherein the control unit comprises a dose adjustment part that is operable by the user to adjust an aerosol output amount.

30. The precision drug administration control method according to claim 28, wherein the drug cartridge comprises an identification device for identifying drug information;
the control unit is configured to obtain the drug information and set an atomization output flow rate and an atomization duration of the drug cartridge based on the drug information.

31. The precision drug administration control method according to claim 24, wherein after the drug cartridge is controlled to stop an atomization operation, the control unit controls the flow generator to shut down with a delay according to a set administration location, to generate an incremental airflow to deliver the aerosol to the administration location in the respiratory tract.

32. The precision drug administration control method according to claim 31, wherein the delay of the flow generator is set to 0.1~5.0 s.
